# EUROPEAN PATENT SPECIFICATION

(11) **EP 2 041 315 B1**
(45) Date of publication and mention of the grant of the patent: **25.08.2010**
(21) Application number: 07809616.1
(22) Date of filing: 14.06.2007
(51) Int. Cl.: C12Q 1/68

(54) **METHODS AND COMPOSITIONS FOR OBTAINING AMPLIFIABLE NUCLEIC ACIDS FROM TISSUES, CELLS, OR VIRUSES EXPOSED TO TRANSPORT MEDIA**
VERFAHREN UND ZUSAMMENSETZUNGEN FÜR DEN ERHALT AMPLIFIZIERBARER NUKLEINSÄUREN AUS TRANSPORTMEDIEN AUSGESETZTEN GEWEBEN, ZELLEN ODER VIREN
PROCÉDÉS ET COMPOSITIONS PERMETTANT D'OBTENIR DES ACIDES NUCLÉIQUES AMPLIFIABLES DE TISSUS, CELLULES, OU VIRUS EXPOSÉS À DES MILIEUX DE TRANSPORT

(30) Priority: 19.06.2006 US 814787 P
(43) Date of publication of application: 01.04.2009
(73) Proprietor: Becton, Dickinson and Company, Franklin Lakes, NJ 07417 (US)
(72) Inventor: MCGILL, Eric, S., Joppa, MD 21085 (US)
(74) Representative: von Kreisler Selting Werner
(86) International application number: PCT/US2007/014125
(87) International publication number: WO 2007/149350

(56) References cited:
- EP-A- 0 511 430
- EP-A- 1 085 098
- WO-A-99/29890
- WO-A-99/31273
- WO-A-03/023062
- WO-A-2005/121373
- US-A1- 2005 032 105
- CATTORETTI G ET AL: "ANTIGEN UNMASKING ON FORMALIN-FIXED, PARAFFIN-EMBEDDED TISSUE SECTIONS" JOURNAL OF PATHOLOGY, CHICHESTER, SUSSEX, GB, vol. 171, 1993, pages 83-98, XP003016419 ISSN: 0022-3417
- CHAN K L ET AL: "Revisiting ischemia and reperfusion injury as a possible cause of necrotizing enterocolitis: Role of nitric oxide and superoxide dismutase." JOURNAL OF PEDIATRIC SURGERY JUN 2002, vol. 37, no. 6, June 2002 (2002-06), pages 828-834, XP002464976 ISSN: 1531-5037
- SHIMA S ET AL: "POLY-L LYSINE PRODUCED BY STREPTOMYCES-ALBULUS 2. TAXONOMY AND FERMENTATION STUDIES" AGRICULTURAL AND BIOLOGICAL CHEMISTRY, vol. 45, no. 11, 1981, pages 2497-2502, XP002464977 ISSN: 0002-1369

## Description

### BACKGROUND OF THE INVENTION

The invention relates generally to methods and compositions for conducting genetic testing on preserved biological samples, and, in particular to performing nucleic acid extraction and amplification methods on such preserved samples.

In the fields of medical diagnosis and medical research, samples (*e.g*., tissues) are taken from a patient or subject (*e.g*. a human patient, a human subject, an experimental animal model of a human disease) to determine the condition of the subject in support of the research, determine the current condition of the patient for making a medical diagnosis, determine the response of the patient to a current course of therapy or treatment, etc.

Samples that are obtained for analysis, either for performing medical diagnosis or for use in scientific research, are often placed in a special transport/preservative medium to keep the sample from degrading or decomposing when removed from the subject. Thus, the sample will as closely as possible be in the exact condition it was in when removed from the subject. This ensures that the sample accurately reflects the state of the patient or subject at the time of sampling and will therefore yield the most accurate result in any subsequent studies of the sample. Some of these studies will involve nucleic acid (for example deoxyribonucleic acid (DNA) or ribonucleic acid (RNA)) extraction and amplification.

Extracting DNA and/or RNA from biological samples requires, among other steps, lysis of the cell wall (in the case of prokaryotic cells), lysis of the cell membrane (in the case of certain eukaryotic cells) or lysis of the viral capsid (in the case of viruses). Subsequent amplification requires, in part the attachment of primers to specific sites within the target nucleic acid.

There are a number of protocols available for the extraction and subsequent amplification of nucleic acid. Some of these protocols utilize high throughput devices. High throughput devices are automatic in the sense that a sample is placed within the device together with appropriate chemicals and the extraction and amplification steps take place without further input from the operator (*e.g*. Viper^{™} System by Becton Dickinson®). Other protocols utilize less sophisticated equipment and are typically referred to as manual procedures. However, regardless of the protocol, the need to lyse the cells or viral capsids in order to release the nucleic acid, the need to attach chemicals such as ferric oxide in order to extract the nucleic acid from the rest of the sample, and the need to attach primers to the target nucleic acid in order to amplify the nucleic acid remain.

Transport media typically contain one or more constituents that preserve certain sample characteristics (*e.g*., prevent the breakdown of the cell wall or the cell membrane by cell lysis). In addition, some of these constituents serve the dual purpose of preservation and decontamination of the sample. Typically, these constituents are either cationic or help preserve the cationic characteristics of the transport media. These constituents are known to interfere with the ability to lyse cell membranes and walls, the ability to attach chemicals utilized in nucleic acid extraction, and the ability to amplify target nucleic acid.

Transport media, such as SurePath® (Tripath Imaging, Inc., N.C.) or PreservCyt® (Cytyc Corp., MA)., adversely affect the ability to extract amplifiable target nucleic acid from samples exposed thereto. Many reasons have been suggested to explain this observed adverse effect including: 1) degradation of the nucleic acids by constituents in the media; 2) chemical alteration of the nucleic acids by constituents in the media; and 3) inhibition of the cell lysing mechanism in the tissue, which inhibits the release the nucleic acids for extraction and amplification

Current methods to overcome the effect of certain constituents of transport media on nucleic acids typically involve additional cheating/cooling cycles and extra centrifugation steps, see e.g. WO 03/023062. Such extra steps are not compatible with many high throughput automatic devices such as, for example, the aforementioned Viper^{™} System. Even in situations where automation is not involved, such steps are nevertheless time-consuming. The additional time required by these steps can delay obtaining the test results and is preferably avoided.

### SUMMARY OF THE INVENTION

The present invention addresses the problems of difficult cell lysis, poor nucleic acid extraction, and poor amplification of target nucleic acid from samples that have been exposed to the preserving/decontaminating constituents in transport media. The present invention employs an additive that allows the desired steps of cell lysis, nucleic acid extraction, and nucleic acid amplification to be performed without the need to resort to heating or extra centrifugation to facilitate/accomplish lysis or amplification.

Disclosed herein is a composition comprising a cationic transport medium and an additive that is a source for anionic species, where the additive substantially compensates for the undesired effects of the preserving/decontaminating constituents contained in the transport medium. The additive allows the cells in the sample to be lysed and the nucleic acids in the sample to be extracted and amplified without the need for the aforementioned additional steps

Further disclosed herein is a method for treating samples that have been exposed to a cationic transport medium comprising the step of adding said additive to the transport medium, either before, at the same time as or after the sample is added.

### BRIEF DESCRIPTION OF THE DRAWINGS

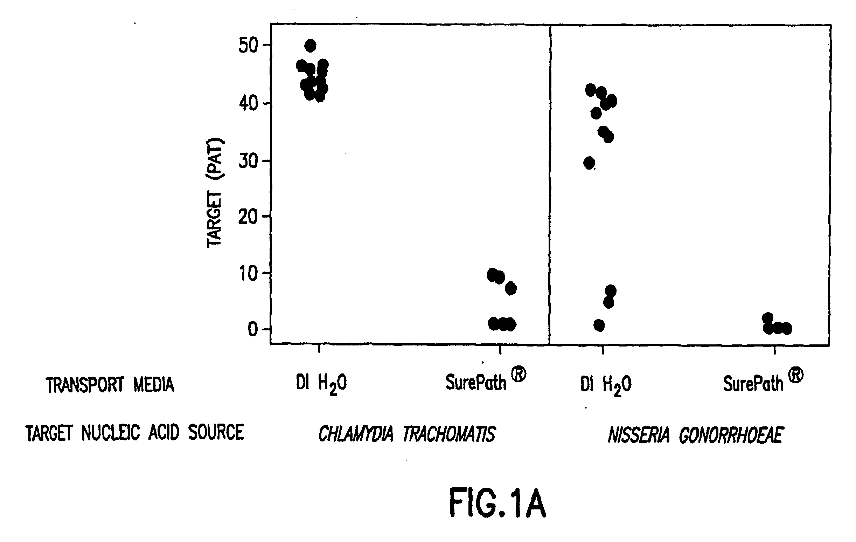

Fig. 1(A)-(B) are graphs that compare the reduced ability to extract and amplify nucleic acid from samples (transport media or deionized water) spiked with target nucleic acid to an increased extraction and amplification of target nucleic acid from similar samples that are further treated with an additive, ferrous sulfate; and

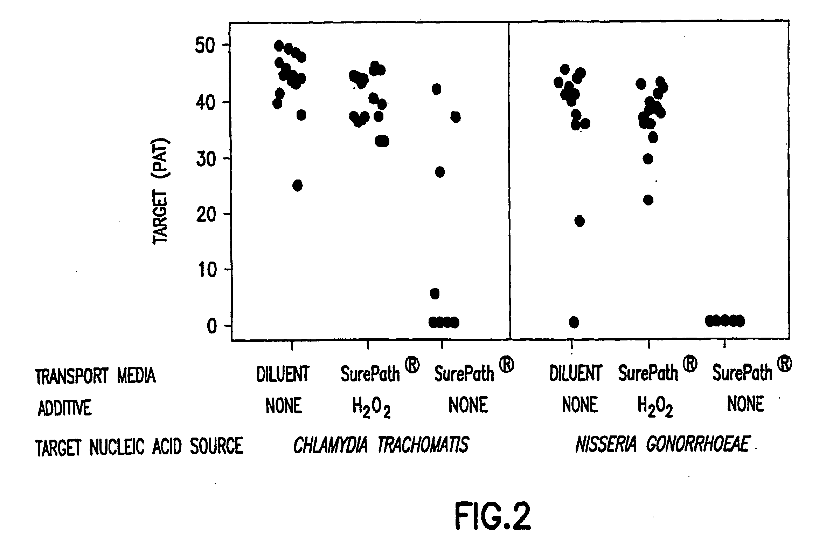

Fig. 2 are graphs that compare the reduced ability to extract and amplify nucleic acid from samples (transport media or diluent) spiked with target nucleic acid to an increased extraction and amplification of target nucleic acid from similar samples that are further treated with an additive, hydrogen peroxide solution.

### DETAILED DESCRIPTION

The present invention is directed to method of treating biological samples that have been in contact with a transport medium so as to neutralize the negative effects of the transport medium and thus facilitate subsequent analyses on said samples, such as nucleic acid extraction and amplification.

Although not wishing to be bound to a particular theory, applicant believes that the adverse effect of transport media upon nucleic acid extraction and amplification is caused by at least one, and possibly both of the following mechanisms. First, cells in the sample exposed to the constituents of the transport media are more refractory to lysis. This increased resistance to lysis inhibits the release of nucleic acid and reduces the amount of nucleic acid available for extraction and amplification. Second, the negatively charge nucleic acid, once it is released, interacts with one or more cationic constituents in the transport medium. This takes up the binding sites on the nucleic acid, blocking the attachment of, for example, ferric oxide or amplification primers, such that extraction and amplification of the nucleic acid is inhibited.

Again not wishing to be held to a particular theory, Applicant believes that an additive of the invention either inhibits the attachment of the cationic constituents in the transport medium to the target nucleic acid or causes the cationic constituents to disengage from the nucleic acid. Under either theory, the nucleic acid has more binding sites for attachment to constituents in the extraction phase and more binding sites for primer attachment during the amplification phase than nucleic acids that have been placed in contact with transport media without the benefit of the additive described herein. In other embodiments, the additive of the present invention is also selected to facilitate cell lysis, thus increasing access to the nucleic acids for extraction and amplification.

With regard to extraction, the additive at least partially compensates for the adverse affects of the transport media constituents that interfere with the ability of the, for example, ferric oxide particles to attach to the target nucleic acid as part of the extraction procedure. With regard to nucleic acid amplification, the additive at least partially compensates for the adverse affects of the transport media on the ability of primers, which are used to amplify nucleic acids (*e.g*., DNA or RNA), to attach to binding sites on the nucleic acid.

For ease in understanding the disclosure, the following definitions are employed.

As used herein, the term "extracting nucleic acid" or "extraction" includes, but is not limited to, the steps of cell lysis (capsid lysis in the case of viral particles) followed by capture of the nucleic acid, separating the nucleic acid from the rest of the sample.

As used herein the term "nucleic acid" refers to either ribonucleic acid (RNA) or deoxyribonucleic acid (DNA) or both RNA and DNA.

As used herein, the term "automated protocol" refers to protocols that employ an automated system for nucleic acid extraction followed by amplification of the nucleic acid. An example of such an automated system is the Viper^{™} System by Becton Dickinson®.

As used herein, the term "manual protocol" refers to protocols that do not use automated systems for nucleic acid extraction and amplification. Manual protocols do not employ strictly manual steps, and may include some automated steps in addition to the manual steps. Both automated protocols and manual protocols are well known to those skilled in the art and can be found in various reference materials such as Molecular Biology techniques Manual, 3rd edition, Coyne, Vernon E. et al., editors.

As used herein, the term "tissue" refers either to a group of cells taken as a unit from, for example, a body organ, or to single cells (*e.g*., cells from vaginal swabs or cell fractions from whole blood, for example, white blood cells).

As used herein, the term "microorganism" refers to either bacteria or viruses.

As used herein the term "sample" alternatively, "biological sample" refers to any sample taken for analysis and includes but is not limited to tissues, groups of cells, single cells (eukaryote or prokaryote) or viruses.

As used herein, the term "transport medium" or the plural "transport media" refers to any medium in which a tissue or biological sample is placed so that the physical and chemical characteristics of the tissue sample are preserved and/or decontamination of the tissue sample is achieved. Further, as used herein, the term transport medium/media is synonymous with storage medium/media.

As used herein, the term "additive" refers to a chemical or chemical composition comprised of or capable of releasing a negative ionic (*i.e*., anionic) species, wherein the additive comprises hydrogen peroxide, sodium peroxide, area-hydrogen peroxide or ferrous sulfate.

Disclosed herein is a composition comprising a cationic transport medium and an anionic additive.

Transport media used for the preservation and/or decontamination of biological samples are well known in the art. These media include, for example, compositions containing formaldehyde, formic acid, methanol, ethanol, formalin, EDTA, cationic polypeptides (*e.g*., polymyxin), cationic poly amino acids (*e.g*., poly-1-lysine), cationic polysaccharides, or a combination thereof. In addition to the examples, one skilled in the art is aware that there are numerous transport media constituents used for the preservation/decontamination of biological samples that yield cationic species. Concentrations of these preserving/decontaminating constituents in transport media are such that decontamination is achieved while adequately preserving the sample and sample components (*e.g*., nucleic acid) for subsequent analysis. Many such media are commercially available.

In one embodiment, the transport medium is 10% buffered formalin. In another embodiment the transport medium is the composition contained in commercially available transport media such as SurePath® or PreservCyt®.

The anionic additive capable of providing an anionic species in the final composition is hydrogen peroxide, sodium peroxide or urea-hydrogen peroxide. Hydrogen peroxide, for example, dissociates in aqueous solution, releasing the anionic species HOO-. In one embodiment, the additive is hydrogen peroxide.

In another embodiment of the invention, the additive is ferrous sulfate added either as an aqueous solution (volume to volume) or a solid (weight to volume).

In creating the composition, the additive is combined with the transport medium either as a solution (volume to volume) or a solid (weight to volume). The amount of additive required is that amount capable of neutralizing the adverse effects of the cationic species without significantly harming the sample. Thus, it is contemplated that the amount of additive required will depend at least somewhat on the type and concentration of cationic species contained within the transport medium.

In one exemplary embodiment, a solution of hydrogen peroxide (typically available in either a 3% or 30% solution) is added to the transport medium so as to achieve a final concentration of about 0.01% to about 3% hydrogen peroxide in the transport media. In another exemplary embodiment, the additive is added as either a solution or solid form of ferrous sulfate so as to achieve a final concentration of about 15 millimoles (mM) to about 30 mM of ferrous sulfate in the transport media.

Finally, a further exemplary embodiment adds the additive, either as solid, liquid or a combination of solids and liquids, of ferrous sulfate so as to achieve a final concentration for the ferrous sulfate of about 15 mM to about 30 mM.

Another aspect of the present invention is a method for treating samples that have been exposed to cationic transport media, either at the time of exposure or after. Specifically, in the present invention, a biological sample is provided. That sample is combined with: 1) a transport medium and 2) an additive as described above. The order of the combining steps is not critical. Thus, an additive can be combined with a transport medium immediately before, contemporaneously with or after a sample is combined with a transport medium. Subsequent to the addition of the additive, the cells in the sample are lysed, the nucleic acid is extracted from the rest of the sample, and those extracted nucleic acids are amplified for further analysis.

Samples may be obtained in a number of medically approved ways from a number of sources. Examples include, but are not limited to, blood-draws, biopsies, swabs and in the case of certain bacterial analyses, from cultures.

The constituents in the transport medium that preserve and/or decontaminate tissues, as well as examples of such media, have been described elsewhere in this disclosure. In an exemplary embodiment, the transport medium is one comprised of the formulations found in commercially available transport media such as SurePath® or PreservCyt®.

Examples of additives that provide anions suitable for use in the present invention have been described elsewhere in this disclosure. As previously noted, the additive may be combined with the transport medium as a solution, in a volume to volume measurement or as a solid, in a weight to volume measurement.

The amount of additive that is needed to treat the sample in order to provide the beneficial effect varies depending upon a number of factors. These include, by way of illustration and not by limitation, the particular additive, the concentration of the additive, the specific type of transport medium, the concentration of cationic constituents within the transport medium, and the amount of transport medium to be so treated. These factors for determining the amount of additive for use in a particular application are not listed in order of importance.

The additive is combined with the transport media in an effective amount. As used herein, an effective amount is an amount sufficient to bring about the beneficial effect (*i.e*. improved sample lysis/ability to adequately amplify nucleic acids in sample exposed to transport medium) without adversely affecting the sample. One skilled in the art can readily ascertain the upper and lower limits for the additive concentration for any given combination of additive, transport medium and sample.

In certain preferred embodiments, the additive both increases the number of binding sites for primer attachment to the nucleic acid (again compared to the number of binding sites available for a nucleic acid exposed to transport media without the additive) and facilitates cell lysis. In a preferred embodiment, the additive is a 3% hydrogen peroxide aqueous solution which is added to transport media to obtain the desired concentration of anionic species in the media identified above.

In alternative embodiments, the additive is also combined with a diluent (diluents are typically buffered solutions with or without detergent and are known to those skilled in the art, an example of such a diluent is that utilized by the Becton Dickinson® ProbeTec™ system) to which a sample, previously exposed to transport medium, is subsequently added. In a preferred embodiment, the additive is added to the transport medium, which contains the sample.

The additive is added as a weight percent or volume percent of the transport medium, as appropriate. In a preferred embodiment, the additive is provided in liquid form as a volume percent of the transport medium.

In one embodiment of the invention the amount of additive is determined by the amount of ionic species required to neutralize the adverse effects of the cationic constituents in the transport media. In preferred embodiments about 75 microliters (µLs) to about 350 µLs of a 3% hydrogen peroxide aqueous solution is added to about 1 milliliter (mL) of transport medium. In one example of the preferred embodiments about 200 µLs of a 3% hydrogen peroxide aqueous solution is added to about 1 mL of SurePath® transport medium.

In a further exemplary embodiment about 4 milligrams (mg) to about 8 mg of ferrous sulfate is added to about 1mL of transport medium, resulting in a final ferrous sulfate concentration of about 15 mM to 30 mM in the transport media. In another exemplary embodiment about 0.29 mg to about 1.45 mg of sodium chloride is added to about 1 mL of transport medium, resulting in a final sodium chloride concentration of about 5 mM to 25 mM.

In yet another embodiment about 4 mg to about 8 mg of ferrous sulfate and about 0.29 mg to about 1.45 mg of sodium chloride is added to about 1 mL of transport medium, resulting in a final ferrous sulfate concentration of about 15 mM to 30 mM and final sodium chloride concentration of about 5 mM to 25 mM.

Protocols for extracting nucleic acids include those utilizing ferrous oxide or commercially available solutions designed for the purpose. These methods are well known to those skilled in the art and are not further described herein.

Protocols for amplifying nucleic acids include the polymerase chain reaction (PCR), reverse transcription polymerase chain reaction (RT-PCR) and strand displacement amplification (SDA). These protocols for nucleic acid amplification are conventional and well known to one skilled in the art. As such, these mechanisms and processes are not discussed in detail here.

The invention as described above in terms of certain embodiments is further understood with reference to the following examples.

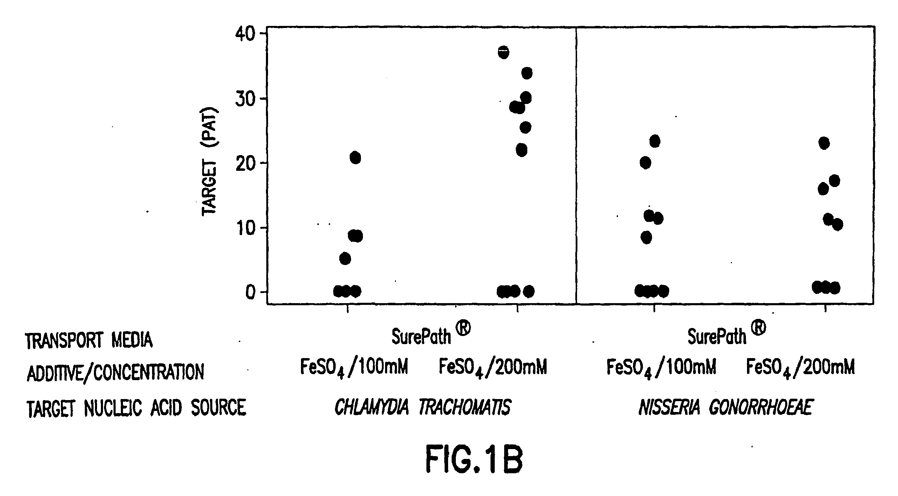

Referring to Fig. 1A and 1B, deionized water (DI H₂O) or transport medium (SurePath®) was spiked with target nucleic acid (the nucleic acid was contained within 200 cells of either *Chlamydia trachomatis* (the infectious elemental bodies) or *Neisseria Gonorrhoeae*). All samples (n=12; represented by the dots in the figure) were prepared for nucleic acid extraction and amplification utilizing the Viper^{™} System by Becton Dickinson®. The spiked samples were incubated at room temperature for 30 minutes. Two mLs of each spiked sample was transferred to individual sample tubes. One sample was further combined with an additive, ferrous sulfate, used at an initial concentration of either 100 mM or 200 mM as combined in the sample.

Referring to Fig. 1, extraction and amplification of target nucleic acid from samples exposed to SurePath® and later treated with ferrous sulfate was greatly enhanced versus recovery from samples not so exposed. Specifically, Figure 1A indicates that very little PAT was observed for cells in transport media. As a control, PAT was obtained for samples of cells in DI H₂O. The PAT for those samples was reasonably high indicating that the samples with media had significantly lower PAT than the samples without. Figure 1B indicates that the value of PAT for the sample with SurePath® media significantly increased when ferrous sulfate (100 mM - 200 mM concentration) was added thereto.

Referring to Fig. 2, clean diluent (DMSO, phosphate, glycerol, and Tween 20) or transport medium (SurePath®) was spiked with target nucleic acid (the nucleic acid contained within 100 cells of *Chlamydia trachomatis* (the infectious elemental bodies) or *Neisseria Gonorrhoeae*). The spiked samples (n=18, represented by the dots in the figure) were incubated at room temperature for 30 minutes. Two mLs of each spiked sample was transferred to individual sample tubes. One sample was then further treated with an additive, a 3% hydrogen peroxide solution, at a concentration of 200 µLs of additive per 1 mL of transport medium. All samples were prepared for nucleic acid extraction and amplification utilizing the Viper^{™} System by Becton Dickinson®.

Referring again to Fig. 2, the results indicate that extraction and amplification of target nucleic acid from samples exposed to SurePath® and later treated with the hydrogen peroxide solution was greatly enhanced versus recovery from samples not so treated.

**Example 1**

The mitigation effect of peroxide in relation to the effect of the transport media on amplification (as measured by PAT) was evaluated. The effect was examined in samples that were both heat lysed and non-heat lysed to determine if the transport media affected lysis. In this example, all samples were prepared for nucleic acid extraction and amplification utilizing the Viper^{™} System by Becton Dickinson®. For this analysis all samples contained clean diluent (DMSO, phosphate, glycerol, and Tween 20), transport medium (SurePath®) and target nucleic acid (the nucleic acid contained either *Chlamydia trachomatis* (the infectious elemental bodies) or *Neisseria Gonorrhoeae*). The samples were divided into two primary groups for analysis. One group was the samples spiked with *Chlamydia trachomatis* (CT). The other group was the samples spiked with *Neisseria Gonorrhoeae* (GC). Each primary group was further subdivided into a first set of samples spiked with 3% hydrogen peroxide solution (0.75 % (by volume) in the sample) and a second set of samples with no peroxide. Samples were yet further subdivided between a first set of samples in which the target nucleic acid was heat lysed and a second set that were not heat lysed. Heat lysis occurred at 114° C. The activity results (PAT) are reported in Table 1 below. A total of twenty four samples were prepared for each evaluation. The distribution of activity (PAT) for each evaluation is reported in Table 1.

**Table 1**

| Target | Pre-Spike Heat Lysis | 0.75 %H₂O₂ | PAT=0 | PAT<10 | PAT<20 | PAT<30 | PAT<40 | PAT>40 |
|---|---|---|---|---|---|---|---|---|
| CT | N | Y | 1 | 1 | 1 | 1 | 10 | 10 |
| CT | N | N | 2 | 4 | 2 | 4 | 8 | 4 |
| CT | Y | Y | 0 | 0 | 0 | 0 | 1 | 23 |
| CT | Y | N | 0 | 0 | 2 | 3 | 6 | 13 |
| GC | N | Y | 0 | 0 | 0 | 0 | 5 | 19 |
| GC | N | N | 10 | 2 | 2 | 1 | 4 | 5 |
| GC | Y | Y | 0 | 0 | 0 | 0 | 2 | 22 |
| GC | Y | N | 8 | 0 | 2 | 4 | 6 | 4 |

As is readily observed, whether or not the samples were heat lysed had negligible effect on PAT results for both CT and GC targets. In contrast, the presence of peroxide had a pronounced effect on the activity, with uniform improvement for both CT and GC targets. That is, samples with peroxide had a markedly higher average PAT compared to samples to which peroxide was not added.

## Claims

1. A method for analyzing samples stored in cationic transport media comprising:
combining the sample with an additive that comprises
- a source for a negatively charged species selected from the group consisting of hydrogen peroxide, sodium peroxide, urea-hydrogen peroxide and ferrous sulfate, and
- a transport media comprising a cationic species;
lysing cells in the sample;
extracting the nucleic acid from the sample; and
amplifying target nucleic acid in the sample.

2. The method of claim 1 wherein the target nucleic acid is DNA or wherein the target nucleic acid is RNA.

3. The method of claim 1 wherein the sample is a blood sample or wherein the sample is cells from a vaginal swab.

4. The method of claim 1 wherein the transport medium has at least one constituent selected from the group consisting of formaldehyde, formic acid, methanol, ethanol, buffered formalin, EDTA, polypeptides, poly amino acids, and polysaccharides.

5. The method of claim 1 wherein the step of extracting is performed by a manual process.

6. The method of claim 1 wherein the step of amplifying step is performed by a manual process.

7. The method of claim 1 wherein the step of extracting and amplifying steps are performed in an automated process.

## Patentansprüche

1. Verfahren zum Analysieren von Proben, die in kationischen Transportmedien gelagert werden, umfassend:
Kombinieren der Probe mit einem Additiv, das Folgendes umfasst:
- eine Quelle für eine negativ geladene Spezies, die aus der Gruppe ausgewählt ist, die aus Wasserstoffperoxid, Natriumperoxid, Harnstoff-Wasserstoffperoxid und Eisen(II)sulfat besteht; und
- ein Transportmedium, das eine kationische Spezies umfasst;
Lysieren von Zellen in der Probe;
Extrahieren der Nucleinsäure aus der Probe; und
Amplifizieren von Target-Nucleinsäure in der Probe.

2. Verfahren gemäß Anspruch 1, wobei es sich bei der Target-Nucleinsäure um DNA handelt oder wobei es sich bei der Target-Nucleinsäure um RNA handelt.

3. Verfahren gemäß Anspruch 1, wobei die Probe eine Blutprobe ist oder wobei es sich bei der Probe um Zellen aus einem Vaginalabstrich handelt.

4. Verfahren gemäß Anspruch 1, wobei das Transportmedium wenigstens einen Bestandteil aufweist, der aus der Gruppe ausgewählt ist, die aus Formaldehyd, Ameisensäure, Methanol, Ethanol, gepuffertem Formalin, EDTA, Polypeptiden, Polyaminosäuren und Polysacchariden besteht.

5. Verfahren gemäß Anspruch 1, wobei der Schritt des Extrahierens mit einem manuellen Verfahren durchgeführt wird.

6. Verfahren gemäß Anspruch 1, wobei der Schritt des Amplifizierens mit einem manuellen Verfahren durchgeführt wird.

7. Verfahren gemäß Anspruch 1, wobei die Schritte des Extrahierens und Amplifizierens in einem automatisierten Verfahren durchgeführt werden.

## Revendications

1. Procédé pour l'analyse d'échantillons stockés dans des milieux de transport cationiques, comprenant les étapes consistant à:
combiner l'échantillon avec un additif comprenant
- une source d'une espèce chargée négativement choisie dans le groupe consistant en peroxyde d'hydrogène, peroxyde de sodium, urée-peroxyde d'hydrogène et sulfate ferreux; et
- un milieu de transport comprenant une espèce cationique;
lyser des cellules dans l'échantillon;
extraire l'acide nucléique à partir de l'échantillon; et
amplifier de l'acide nucléique cible dans l'échantillon.

2. Procédé selon la revendication 1, dans lequel l'acide nucléique cible est de l'ADN ou dans lequel l'acide nucléique cible est de l'ARN.

3. Procédé selon la revendication 1, dans lequel l'échantillon est un échantillon de sang ou dans lequel l'échantillon est des cellules provenant d'un prélèvement vaginal.

4. Procédé selon la revendication 1, dans lequel le milieu de transport comprend au moins un constituant choisi dans le groupe consistant en formaldéhyde, acide formique, méthanol, éthanol, formol tamponné, EDTA, polypeptides, acides polyaminés et polysaccharides.

5. Procédé selon la revendication 1, dans lequel l'étape consistant à extraire est effectuée par un procédé manuel.

6. Procédé selon la revendication 1, dans lequel l'étape consistant à amplifier est effectuée par un procédé manuel.

7. Procédé selon la revendication 1, dans lequel les étapes consistant à extraire et à amplifier sont effectuées dans un procédé automatisé.
